# EUROPEAN PATENT APPLICATION

(11) **EP 0 750 919 A2**
(43) Date of publication of application: **02.01.1997**
(21) Application number: 96304756.8
(22) Date of filing: 27.06.1996
(51) Int. Cl.: A61M 25/10

(54) **Indwelling arterial perfusion ballon catheter**

(30) Priority: 29.06.1995 JP 163628/95
(71) Applicant: FUJI SYSTEMS CORPORATION, Bunkyo-ku, Tokyo (JP)
(72) Inventor: Higami, Tetsuya, Kobe-shi, Hyogo (JP); Ugawa, Junichi, Fuji Sys. Corp. Res. Cent., Yukohama-shi, Kanagawa (JP)
(74) Representative: Leale, Robin George

(57) **Abstract**

An intra-vascular dwelling balloon catheter for arterial perfusion, for use in operations for aortic aneurysm for example, comprises a flexible elongate basic body (2) having a balloon (6) attached to a tip section (5). An axial bore (10) with its front end closed and its rear end open is formed in the catheter body of the catheter to extend rearwardly of the balloon. One or more side holes (12) for perfusing blood are provided in the body near the rear end of the balloon, and a rod (14) is removably received in the said bore from the open rear end thereof so that the side hole is closed. Ribs (7) are provided on the external surface of the balloon.

## Description

The present invention relates to an intra-vascular indwelling arterial perfusion balloon catheter for use in, for example, operations for aortic aneurysm.

Of operations for aortic aneurysm, an operation for aneurysm in aorta including the aortic arch is still technically very difficult. The procedure is performed while blood flow in the aortic arch is completely interrupted. To completely interrupt blood flow in the aortic arch, cardiac pulsation is stopped according to the conventional method for inducing cardiac arrest. In that case, however, blood supply to the brain as well as to the visceral organs in the chest and below (including the liver, kidney, and intestines) through the aortic arch is stopped simultaneously, which kills tissue of the organs because of ischemia. For this reason, in an operation for aortic arch aneurysm, it is necessary to take steps for specific cerebral protection and visceral protection in the chest and below. Known protection strategies involve ultra hypothermic circulatory arrest, and selective organ perfusion. The hypothermic circulatory arrest method is technically convenient, but has time limitation. The perfusion method provides satisfactory organ preservation against ischemia, but is troublesome because it requires another set of infusion lines. Thus each method has some advantages as well as some disadvantages. In recent years the organ perfusion method has been used in most cases.

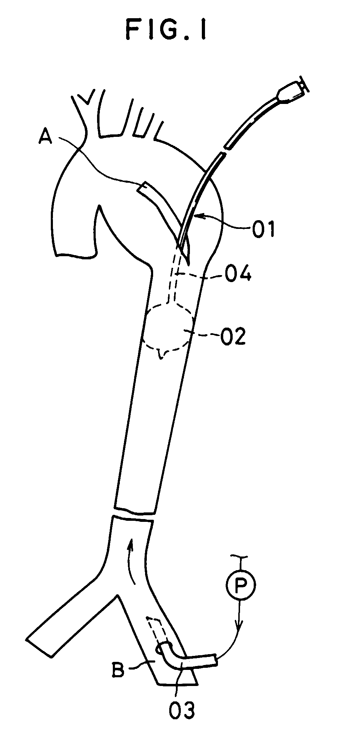

When the perfusion method is applied to various organs in the chest or below during arch reconstruction, conventionally the operation is performed in two steps. Referring to Fig. 1, a balloon catheters 01 for aortic obstruction is inserted through the operative field, namely the incised aortic arch involving aneurysm A, toward the distal side, and the balloon 02 is expanded to obstruct the aorta. At the same time an arterial cannula 03 is inserted through the femoral artery B, and arterial blood is supplied thereto. A conventional balloon catheter 01 has a configuration in which a balloon 02 is attached to a shaft 04 of a single lumen made of polychloride resin. However, as the balloon catheter is inserted through the operative field, namely the incised aortic arch involving aneurysm A, the balloon catheter itself may become an obstacle to the operation, which makes it difficult to perform the arch reconstruction. Also, as the conventional type of balloon catheter 01 is used to obstruct the descending aorta from the proximal side, the catheter is pushed up by blood flow therein from the distal side, which has the disadvantage of making it easier for the catheter to slip out from its dwelling position.

According to the present invention there is provided an intra-vascular dwelling balloon catheter for arterial perfusion, comprising an elongate flexible body having a balloon attached to a tip section thereof, an inner bore with its front end closed and its rear end open being formed in the said body and extending rearwardly of the balloon, at least one side hole for perfusing blood communicating with said inner bore being formed in the said body near to the rear end of the balloon, a rod being detachably engaged in the said inner bore so that it closes the or each said side hole, and ribs being formed on the external surface of the balloon.

In one form of the invention, the front end of the said inner bore is closed at a position near the rear end of the balloon, and the said tip section of the body of the catheter is of smaller diameter than the body in general.

In another form of the invention the front end of the said inner bore is closed at a position near the front end of the balloon, and the said tip section of the body of the catheter is of substantially the same diameter as the body in general.

Preferably the said ribs are provided in a mesh-like form over the entire surface of the balloon so that the ribs do not cross the center line of the body of the catheter at right angles. The ribs preferably comprise a first section in which the ribs are each inclined at an acute angle relative to the center line of the catheter body and a second section in which the ribs are inclined at an obtuse angle relative to the center line so that they cross the ribs of the first section.

Preferably, a plurality of said side holes are provided, spaced lengthwise of the said body.

Preferably an axial passage, through which a guide wire can be inserted, is provided in the said rod, and a further axial passage for a said guide wire is provided in the tip section of the body to communicate colinearly with the axial passage in the rod. A valve to stop bleeding is then provided to close the said passage in the tip section of the catheter body, a slit for the passage of a said guide wire being formed in the valve.

Preferably the basic body of the catheter is radiopaque.

Preferably a side hole for measuring blood pressure is provided in the basic body of the catheter.

A catheter according to the invention only has to be inserted at one location, so that the problem of interfering with the operative field, as observed in conventional technology, can be avoided. Also, the said ribs provided on the external surface of the balloon prevent the balloon from slipping out of its desired location. If, as is preferred, the ribs are of meshlike form, the frictional resistance between the balloon and an internal wall of the blood vessel is increased, so that the balloon is even more securely held in position, whereby an operation can be executed under safe conditions and also simplified. Further, when an axial passage through which a guide wire can be inserted is provided in the said rod as well as in the tip section of the catheter body, it becomes possible to insert the body of the catheter along the guide wire, which makes insertion easier.

Some embodiments of the invention will now be described by way of example and with reference to the accompanying drawings, in which:-
Fig. 1 is an explanatory view to illustrate the effects of using a conventional type of balloon catheter;
Fig. 2 is a front view showing an intra-vascular dwelling balloon catheter for arterial perfusion according to one embodiment of the present invention, with a portion thereof cut and part of the length thereof in the axial direction omitted;
Fig. 3 is an enlarged cross-sectional view of the balloon catheter taken along the line III-III in Fig. 2;
Fig. 4 is a partial view showing an enlarged view of a portion of a balloon before being expanded;
Fig. 5 is a enlarged partial cross-sectional view taken along the line V-V in Fig. 4;
Fig. 6A, Fig. 6B, and Fig. 6C are side views of respective forms of slit in the valve to stop bleeding;
Fig. 7 is an explanatory view to illustrate the use of the embodiment; and
Fig. 8 is a front view showing a catheter according to another embodiment of the invention, with a portion thereof cut and part of the length thereof in the axial direction omitted.

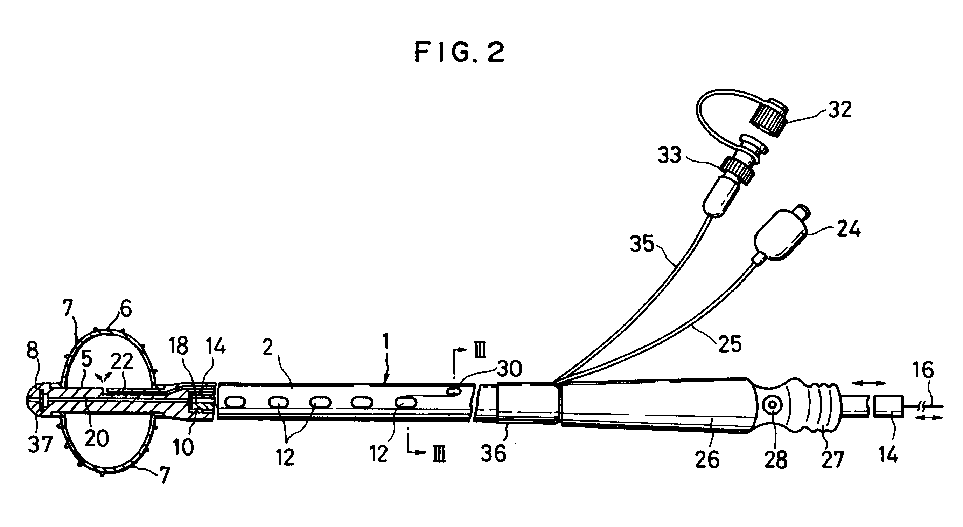

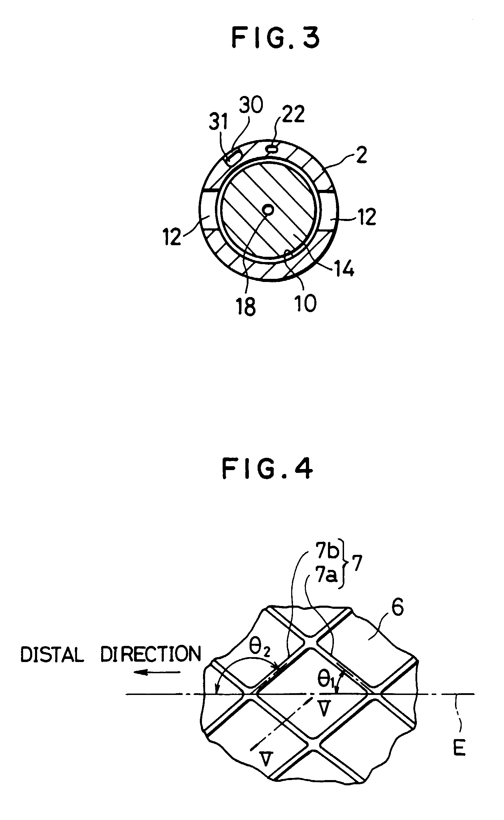

In Figs. 2 and 3, reference 1 indicates an intra-vascular dwelling balloon catheter for arterial perfusion, having a basic body 2 formed as a whole into a flexible form, having a prespecified length, and made of a material such as polyvinylchloride resin, of a material such as polyvinylchloride resin, polyurethane resin, or silicon rubber. This basic body 2 of the catheter is made of an appropriate material and is radiopaque. Formed in a tip section of the basic body 2 of the catheter is a small diameter section 5, and a balloon 6 made of silicon rubber is attached to the small diameter section. The balloon 6 closely embraces the small diameter section 5 before it is expanded, and the diameter when so embracing the small diameter section is substantially the same as that of the body section other than the small diameter section. Reference numeral 7 indicates a rib for dwelling and fixing provided on an external surface of the balloon 6. This rib 7 may be formed by providing a number of small projections or the like, of by providing projecting strips or the like crossing each other.

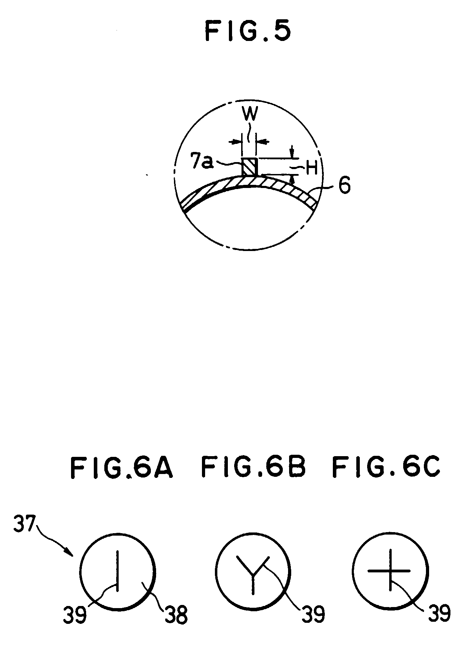

An example of the rib 7 formed by providing projecting strips or the like crossing each other is shown in Figs. 4 and 5. This rib 7 is provided in a mesh-like form over the entire surface of the balloon 6, but so that none of the ribs crosses the center line E of the basic body 2 of the catheter. Namely the ribs 7 comprises a first portion 7a in which a plurality of ribs are provided at a predetermined spacing from each other and each inclined at a predetermined acute angle θ₁ in the distal direction relative to the center line E of the basic body 2 of the catheter, and a second section 7b in which a plurality of ribs are provided at a predetermined spacing from each other and each inclined at an obtuse angle θ₂ in the direction opposite to the distal direction relative to the center line E. The slope angles of the two sections 7a, 7b relative to the center line E of the body 2 are symmetrical around the crossing point. The ribs 7 generate resistance against any force tending to rotate the balloon 6 around the axis E, and they also increase the contact and frictional resistance between the balloon 6 and an length of the balloon 6 in the peripheral direction in the sections 7a and 7b, based on the slope angles as described above. In addition, the cross-sectional shape of the sections 7a, 7b is substantially square or rectangular. As for size of the two sections 7a, 7b, as shown by the section 7a in Fig. 5, when the balloon 6 is not expanded the width W is in a range from 0.1 to 3.0 mm, and preferably in a range from 0.2 mm to 0.7 mm, and the height H is in a range from 0.1 mm to 2.0 mm, and preferably in a range from 0.2 mm to 0.5 mm. Also the difference between a convex section and a concave section, in a case where the external surface of the balloon 6 is irregular, is in a range from 0.1 to 2.0 mm, and preferably in a range from 0.2 mm to 0.5 mm. Furthermore the cross-sectional shape of the sections 7a, 7b should preferably be square or rectangular having sharp edges, as shown in Fig. 5, because then the frictional resistance between the balloon and an internal wall of a blood vessel becomes larger. However, the cross-sectional shape is not necessarily limited to that described above.

The balloon 6 having ribs 7 as described above is made by, for example, using core pins each having a mesh-like concave section on the surface as core pins for a die (not shown in the figures), press-molding the balloon with silicon rubber, taking the balloon out of the press die, and then turning it inside out. Alternatively the balloon can be made by forming the same fine mesh-like concave sections on a surface of a balloon molding section of a press die and pressing the silicon rubber as described above. Of course, in this case it is not necessary to turn the balloon inside out.

In Fig. 2, reference 8 indicates a part-spherical section formed at the tip of the small diameter section 5. An inner bore 10, with its front end terminating at a position near the rear end of the balloon 6, and its rear end open, is formed in the body of the catheter 2 rearwardly of the balloon 6. A plurality of side holes 12 each communicating with the inner bore 10 are formed in the basic body 2, spaced axially thereof. The side holes are used for perfusing blood. It is not necessary to provide a plurality of side holes; only one such hole need be provided. A stiletto-like rod 14 made of a material such as polyvinylchloride resin, polyurethane resin, or polyethylene, which gives resilience to the body 2, is removably received in the bore 10 from its open rear end, so that the external surface of the rod closes the side holes 12. An axial passage 18, through which a guide wire 16 can be inserted, is formed in the rod 14. Also, in the distal direction of the body 2 of the catheter, including the small diameter section 5 and the spherical section 8, into which the bore 10 does not extend, there is an axial passage 20 communicating coaxially with passage 18 in the rod 14, through which the guide wire 16 can be inserted. Reference 22 indicates a lumen for expanding balloon 6, and a branch tube 25 with a one-way valve 24 is connected to the lumen. Reference 26 indicates a connector having a larger diameter provided at the rear end of the body 2 of the catheter, and a connecting section 27 for connecting a tube for blood infusion (not shown) is provided. Reference 28 indicates an air-vent for ventilating air when infusing blood. Reference 30 indicates a side hole formed in the body 2 and communicating with a lumen 31. A branch tube 35 for measurement of blood pressure, having a lure connector 33 with a cap 32, is connected to the lumen 31 from the outside. Reference 36 indicates a cover tube for protecting the tubes 35,25 where they join the body. Reference 37 indicates a silicone rubber valve to stop bleeding, provided in the tip section of the axial passage 20 near to the section 8, and formed with a slit 39 for inserting the guide wire 16 therethrough, at a central position of a basic body 38 of disk form, as shown in Fig. 6A. Fig. 6B and Fig. 6C show respective modifications of the slit 39. The valve 37 prevents blood from flowing out of the end of the axial passage 20.

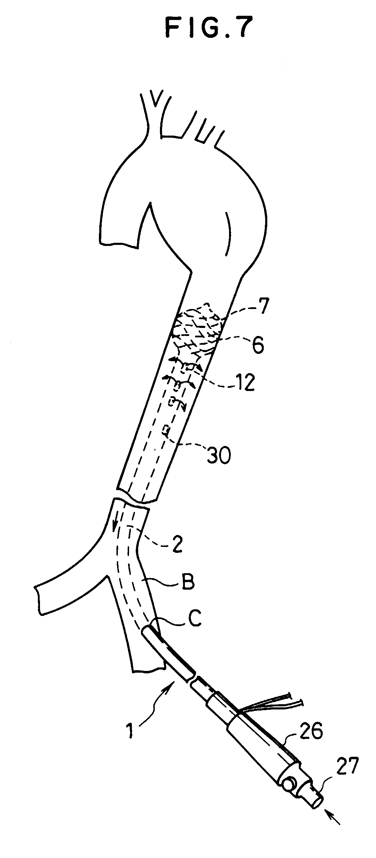

The manner of use of the catheter 1 will now be described with reference to Fig. 7.

To locate the balloon catheter 1 so that the balloon 6 is positioned in the descending aorta at a position near the operative field, the basic body 2 of the catheter, with the rod 14 engaged in the inner bore 10, is inserted from an incised section opened in the femoral artery B, so-called regressively along the guide wire 16 which has been inserted previously, and when the body 2 of the catheter reaches a desired position it is fixed by expanding the balloon 6. By this means, the descending aorta is obstructed on the inside, and the balloon catheter 1 is located and fixed as shown in Fig. 7. In this state, because of the ribs 7 provided on the external surface of the balloon, the frictional contact between the balloon and the internal wall of the blood vessel is increased and the balloon presses into the internal wall of the descending aorta, so that the catheter is very unlikely to slip from its desired position.

After the catheter has been located and fixed, the rod 14 is pulled out of the bore 10 of the body 2 of the catheter. Blood is then infused, after any air in the blood infusion line has been ventilated through the air vent 28. The blood flows from the tube 25 into the bore 10 in the body 2 and thence via the side holes 12 into the descending aorta, from where it is sent to the arteries in the visceral organs in the chest and below, as well as femoral artery B.

As previously described, in the conventional technology a balloon catheter 01 for blocking the aorta, and an arterial cannula 03, are required to be inserted from different places. The balloon catheter 1 according to the present invention, on the other hand, has only to be inserted from one place, which makes it possible to eliminate interference in the operative field by the balloon catheter, and for this reason an operation for aortic aneurysm at the arch section thereof becomes quite easy.

Description will now be made concerning the lack of tendency of the balloon catheter to slip from its located position, with reference to results of the experiments described below.

The balloon 6 of a 14 Fr balloon catheter l was inserted about 3 cm into an artery of the carotid artery section having an inner diameter of around 8 mm, which artery was removed from a cadaver 1 to 2 hours after death and one edge thereof fixed onto a base for experiment. About 2 ml of sterilized water was injected with a syringe through the one-way valve 24 to expand the balloon, and the internal pressure in the balloon was adjusted to 400 mmHg to fix the balloon catheter in the blood vessel. One end of a 200g tension gauge was attached to the connector 26 of the balloon catheter, the other edge of the tension gauge was held with the fingers and gently pulled, the maximum load just before the balloon 6 slipped relative to the blood vessel was measured 3 times under the same conditions, and an average value was obtained. Then, using two conventional types of balloon catheter each having the same size as that of this balloon catheter 1, one of a linear type in which a partially linear rib is provided on the external surface of the balloon and the other of a dot type in which a number of dot-formed ribs are provided, the same experiment was performed, and again the maximum values were measured and the averages calculated. The results are shown in the table below.

**Table**

| Times Type | 1 | 2 | 3 | Average value |
|---|---|---|---|---|
| A | 75g | 75g | 70g | 73g |
| B | 45g | 40g | 40 | 42g |
| C | 115g | 105g | 110g | 110g |
| A = linear type of catheter | | | | |
| B = dot type of catheter | | | | |
| C = mesh-like type of catheter | | | | |

As these results clearly indicate, in the balloon catheter 1 having mesh-like ribs 7 the tendency to slip relative to the internal wall of the blood vessel was the lowest, and thus the balloon catheter 1 can be reliably located inside a blood vessel. The reason for this has not yet been clarified, but possibly it is that the length in the peripheral direction of the balloon 6 in the two sections 7a,7b, which do not cross the center line E at right angles but are inclined thereto, is large as compared with that of the equivalent section of a conventional balloon catheter of the linear rib type or dot type, and furthermore the crossing point between the two sections 7a and 7b shows resistance against a force directed around the axis of the balloon, whereby the frictional contact becomes larger as a whole.

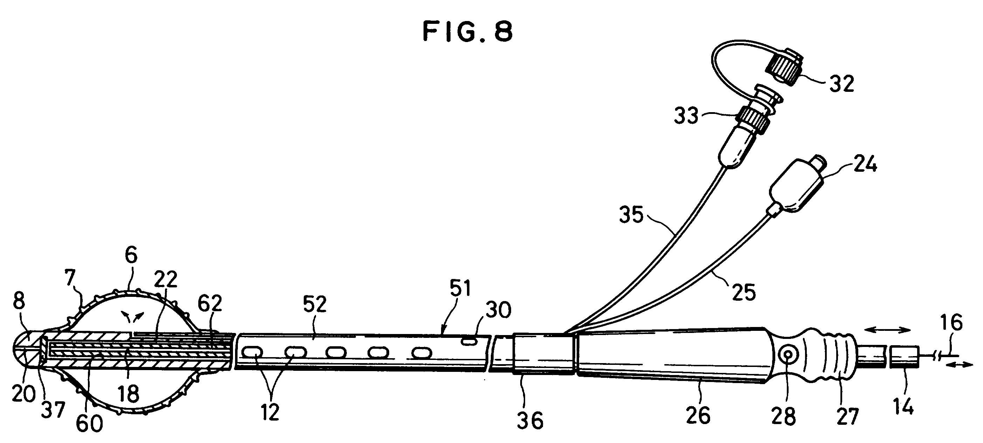

Fig. 8 shows another embodiment of the invention. The balloon catheter 51 of this embodiment has the front end of an inner bore 60 formed in a basic body 52 of the catheter terminating at a position near a front end of the balloon 6. A rod 62 is engaged in the bore 60, and a tip section of the body 52 at the location of the balloon 6, has substantially same diameter as that of the body 52. Other parts are the same as in the previous embodiment.

## Claims

1. An intra-vascular dwelling balloon catheter for arterial perfusion, comprising an elongate flexible body (2) having a balloon (6) attached to a tip section (5) thereof, an inner bore (10) with its front end closed and its rear end open being formed in the said body and extending rearwardly of the balloon, at least one side hole (12) for perfusing blood communicating with said inner bore being formed in the said body near to the rear end of the balloon, a rod (14) being detachably engaged in the said inner bore so that it closes the or each said side hole, and ribs (7) being formed on the external surface of the balloon.

2. A catheter according to claim 1, wherein the front end of the said inner bore (10) is closed at a position near the rear end of the balloon (6), and the said tip section (5) of the said body of the catheter is of smaller diameter than the body in general.

3. A catheter according to claim 1, wherein the front end of the said inner bore (60) is closed at a position near the front end of the balloon (6), and the said tip section of the body (52) of the catheter is of substantially the same diameter as the body in general.

4. A catheter according to any of claims 1 to 3, wherein the said ribs (7) are provided over the entire surface of the balloon in a mesh-like form so that the ribs do not cross the center line (E) of the basic body (2) of the catheter at right angles.

5. A catheter according to claim 4, wherein the said ribs (7) comprise a first section in which the ribs (7a) are each inclined at an acute angle (θ₁) in the distal direction relative to the center line (E) of the body (2) of the catheter, and a second section in which the ribs (7b) are each inclined at an obtuse angle (θ₂) in the distal direction relative to the said center line so that they cross the ribs of the first section.

6. A catheter according to any preceding claim, wherein a plurality of said side holes (12) are provided, spaced lengthwise of the said body (2).

7. A catheter according to any preceding claim, wherein an axial passage (18), through which a guide wire (16) can be inserted, is provided in the said rod (14), and a further axial passage (20), through which a guide wire (16) can be inserted, is provided in the distal side of the said body (2) to communicate with the axial passage in the said rod.

8. A catheter according to claim 7, wherein a valve (37) to stop bleeding is provided to close the said axial passage (20) in the distal side of the body (2), a slit (30) for the passage of a said guide wire being formed in the said valve.

9. A catheter according to any preceding claim, wherein the said body (2) of the catheter is radiopaque.

10. A catheter according to any preceding claim, wherein a side hole (30) for measuring blood pressure is provided in the said body (2) of the catheter.
